# EUROPEAN PATENT SPECIFICATION

(11) **EP 2 242 483 B1**
(45) Date of publication and mention of the grant of the patent: **20.02.2013**
(21) Application number: 08865203.7
(22) Date of filing: 18.12.2008
(51) Int. Cl.: A61K 9/20, A61K 31/4535

(54) **RALOXIFENE COMPOSITION**
RALOXIFEN-ZUSAMMENSETZUNG
COMPOSITION DE RALOXIFÈNE

(30) Priority: 21.12.2007 US 9027
(43) Date of publication of application: 27.10.2010
(73) Proprietor: Synthon B.V., 6545 CM Nijmegen (NL)
(72) Inventor: JANSEN, Korinde Annemarie, 6924 AB Loo (NL)
(74) Representative: van den Broek, Ludovicus A.G.M.
(86) International application number: PCT/EP2008/011102
(87) International publication number: WO 2009/080364

(56) References cited:
- EP-A- 0 670 162
- WO-A-97/35571
- WO-A-2006/052254

## Description

### BACKGROUND OF THE INVENTION

Raloxifene, or (6-hydroxy-2-(4-hydroxy-phenyl)benzo[b]thien-3-yl)-(4-(2-(1-piperidinyl)ethoxy)phenyl) methanone of the formula 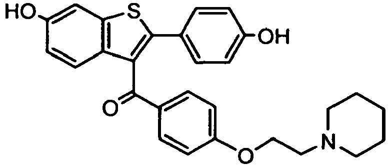 is a pharmaceutically active compound, indicated for treatment and/or prevention of osteoporosis in women. In pharmaceutical formulations, it is used in the form of a hydrochloride salt. Pharmaceutical formulations comprising raloxifene hydrochloride are marketed, e.g., under the brand name Evista® by Eli Lilly.

The raloxifene compound as well as the raloxifene hydrochloride compound have been disclosed, e.g., in US 4418068. Crystalline raloxifene hydrochloride has been disclosed in WO 96/09045 (US 5731327). Raloxifene hydrochloride is very slightly soluble in water (627 µg/ml) as well as in gastric and intestinal fluid. Raloxifene is absorbed rapidly after oral administration and has an absolute bioavailability of only about 2%. Many pharmaceutical formulations of raloxifene and raloxifene hydrochloride are known in the art.

The marketed formulation is a film-coated tablet comprising 60 mg of raloxifene hydrochloride. The excipients in the composition include povidone, polysorbate 80, anhydrous lactose, lactose monohydrate, crospovidone, magnesium stearate, hypromellose, macrogol 400 (polyethylene glycol), carnauba wax, modified pharmaceutical glaze, and propylene glycol.

EP 670162 B1 (US 5811120, US 5972383) discloses an orally administrable pharmaceutical formulation comprising raloxifene or a pharmaceutically acceptable salt thereof (preferably hydrochloride) in combination with (i) a surfactant (most preferably a polyoxyethylene sorbitan fatty acid ester such as polysorbate 80), (ii) a water-soluble diluent (most preferably lactose), and (iii) optionally a hydrophilic binder (most preferably polyvinylpyrrolidone). The formulation can also contain a lubricant (preferably magnesium stearate) and a disintegrant (preferably a cross-linked polymer, more preferably cross-linked polyvinylpyrrolidone). While disclosed as "optional," every claim of EP 670162 B1, US 5811120, and US 5972383 requires the presence of polyvinylpyrrolidone in the pharmaceutical formulation.

EP 781555 A I discloses pharmaceutical unit dosage forms, such as tablets, comprising from 50-200 mg of a benzothiophene, preferably raloxifene hydrochloride. Such dosage amount is taught to be advantageous for treatment or inhibition of bone loss.

Because bioavailability of raloxifene hydrochloride is quite low, attempts have been made to increase bioavailability in pharmaceutical formulations. One attempt comprises improving the physical form of the active substance. WO 97/35571 (US 6458811, US 6797719, US 6894064) discloses a microparticulate form of raloxifene hydrochloride with enhanced bioavailability. Alternatively, EP 826682 B1 discloses amorphous raloxifene hydrochloride with enhanced bioavailability.

Another strategy for improving bioavailability comprises improving the pharmaceutical formulation containing raloxifene hydrochloride. CN 1615860 discloses a composition comprising raloxifene hydrochloride (35-45%), diluent (50-60%), disintegrant (2-4%), lubricant (0.5-1%), and adhesive (2-3%). WO 2006/052254 A2 discloses a compressed pharmaceutical formulation comprising raloxifene hydrochloride and starch in the amount of greater than about 25 %.

Raloxifene hydrochloride is a degradation-sensitive compound, even in tablet formulations. Hartauer et al. (Pharmaceutical Development and Technology, 5(3), 303-310 (2000)) studied the influence of excipients and found that raloxifene hydrochloride is prone to undesirable oxidation in the presence of povidone and crospovidone. Therefore, a specific purity control on these excipients must be done in the course of manufacture. Raloxifene hydrochloride is also prone to aerobic biodegradation, forming impurities such as the raloxifene dimmer (see V. Toader, X. Xu, A. Nicolescu, L. Yu, J.L. Bolton, G.R.J. Thatcher, Chem.Res.Toxicol., 2003, vol. 16, 1264-1276) and the raloxifene N-oxide. In particular, peroxide compounds present in commercial grade povidone (polyvinylpyrrolidone) and crospovidone (cross-linked polyvinylpyrolidone) could form N-oxides of raloxifene and therefore compromise the stability of the pharmaceutical formulation of raloxifene.

An alternative pharmaceutical composition containing raloxifene hydrochloride, preferably a composition that does not contain povidone or starch, is desirable.

### SUMMARY OF THE INVENTION

The present invention relates to a raloxifene composition. Accordingly a first aspect of the invention relates to a pharmaceutical composition, comprising: a) raloxifene or a pharmaceutically acceptable salt thereof, preferably raloxifene hydrochloride, in an amount of 20 to 30 weight %; b) a mixed cellulose excipient in an amount of 25 to 40 weight %, preferably 30 to 35 weight %, said mixed cellulose excipient containing 90 to 95 weight % microcrystalline cellulose and 5 to 10 weight % hydroxypropyl cellulose with respect to the total mass of the mixed cellulose excipient; and c) a disintegrant in an amount of 5 to 12 weight %. The composition preferably does not contain povidone and/or starch. Further excipients normally include a sugar such as lactose. The composition is generally a tablet, especially an immediate release tablet.

### BRIEF DESCRIPTION OF THE DRAWINGS

Figure 1 shows the dissolutions profile of tablets made according to the invention, measured at 1000 ml 0.1% Tween 80 solution.

Figure 2 shows the dissolutions profile of tablets made according to the invention, measured at 1000 ml acetate buffer pH 4.5.

### DETAILED DESCRIPTION OF THE INVENTION

It was found that suitable results in stability studies may be obtained by replacing the prior art's povidone binder with a mixed cellulose excipient in a pharmaceutical composition containing raloxifene hydrochloride. The formation of impurities of raloxifene, e.g. raloxifene-N-oxide and raloxifene dimer, was minimized in comparison with a raloxifene composition containing povidone as a binder/excipient. Indeed, the stability was successful even though crosspovidone was used as a disintegrant. Thus the formulation of the present invention represents another viable composition for providing raloxifene or its salts.

Accordingly, the present invention relates to a pharmaceutical composition comprising raloxifene or a pharmaceutically acceptable salt thereof, a mixed cellulose excipient, and a disintegrant. In a preferred embodiment, the composition contains the hydrochloride salt of raloxifene, i.e., raloxifene hydrochloride. The raloxifene hydrochloride is typically present in an amount of 20-30 weight % of the total weight of the composition. The raloxifene hydrochloride can be used in the solid, crystalline or amorphous, state. Preferably, the raloxifene hydrochloride is in a form of particles having a mean particle size of 5 and 20 microns. In particular, it is desirable that 95% of the raloxifene hydrochloride particles are smaller than 50 microns.

The "mixed cellulose excipient" that serves as a binder system in the present invention is a mixture of microcrystalline cellulose (MCC) and hydroxypropylcellulose (HPC). The term "mixture" merely requires the presence of MCC and HPC together, but does not require any specific degree of mixing nor does it require any particular physical interaction between the MCC and HPC. The mixed cellulose excipient is typically present in an amount of 25 to 40 % of the total mass of the composition, more typically 30-35 weight %. The mixed cellulose excipient is mostly MCC with only a minor portion of HPC, generally 90 to 95 weight % of the MCC and 5 to 10 weight % of the HPC, with respect to the total mass of the mixed cellulose excipient. That is, the ratio of the microcrystalline cellulose and hydroxypropylcellulose in the cellulose excipient is typically from 19 : 1 to 9 : 1(w/w), though is not strictly limited thereto.

The mixed cellulose excipient consists of two kinds of cellulose-type compounds with different properties: the microcrystalline cellulose is water insoluble, while hydroxypropylcellulose is water soluble. This particular combination, in the mutual ratio specified above, has good binding, granulating, and tabletting properties, which makes it suitable for formulating the composition into compressed formulations (dosage forms) by a process of granulation and/or compression. The mixed cellulose excipient is particularly advantageous for a wet granulation process as it minimizes agglomeration of raloxifene particles into large agglomerates, which agglomerates are generally less rapidly dissolving and can provide other processing inconveniences.

The disintegrant is necessary to insure disintegration of the compressed formulation after contacting an aqueous environment such as stomach fluid after oral administration. The amount of the disintegrant is generally from 5 to 12 weight %, based on the total mass of the composition and/or tablet core. The disintegrant may be any conventional disintegrant, however, the use of crospovidone as a disintegrant should be avoided when the composition contains povidone.

The composition of the present invention may also contain other excipients. These include diluents or fillers such as a sugar (e.g., lactose especially lactose monohydrate, a polysaccharide, a cyclodextrin, etc.), a surface active agent (e.g., a Polysorbate (polyoxyethylene sorbitan fatty acid ester), polyethylene glycol-15-hydroxystearate, etc.) and a lubricant (e.g., talc, magnesium stearate, etc). Water soluble fillers are generally preferred. While additional excipients are permitted, in a most preferred embodiment, however, the composition of the present invention does not contain povidone or starch.

The composition of the present invention may be advantageously used in medicine, preferably for the treatment and/or prevention of osteoporosis. In particular, they may be used in a compressed formulation (dosage form), e.g. in a tablet. The compressed formulation contains a therapeutically useful amount of raloxifene, preferably raloxifene hydrochloride, e.g., from 10 to 100 mg of the raloxifene. The nature and amounts of the remaining components should be selected in order to obtain or maintain suitable physical parameters of the blend, good tabletting properties, and/or good granulation properties of the composition. In a preferred embodiment, the compressed formulation is an immediate release compressed formulation (a tablet) having a disintegration time of less than 5 minutes in each of water and a simulated gastric fluid. More precisely, the composition is preferably an immediate release tablet meaning that at least 75% and generally at least 90% of the raloxifene is released/dissolved within 30 minutes in an appropriate dissolution test. An appropriate test is readily determined by workers skilled in the pharmaceutical arts following the usual conditions and procedures that would be required by the, e.g., U.S. FDA. For example, tablets of the invention typically have a dissolution profile (USP apparatus II) such that more than 90% raloxifene is dissolved in 30 minutes in water or acetate buffer (pH=4.5) using a rotation speed of 50 rpm in peak vessels at a temperature of 37.5°C.

In essence, the composition may be formulated into the dosage form or tablet by any the general procedures, which comprise a wet granulation, a dry granulation or a direct compression. The wet granulation process is preferred. In this process, water is advantageously used as the wetting medium. In an example of the wet granulation process, the pre-weighed amounts of the active substance (raloxifene hydrochloride), the mixed cellulose excipient, the filler, the surfactant and (at least a part of) the disintegrant are mixed with a suitable amount of water to make a wet mass which is then granulated into granulate particles that are dried and screened to the desired size. Alternatively, the HPC can be pre-dissolved in the granulation medium optionally with the surfactant as well. The water is then combined with the raloxifene, the MCC and remaining granulate excipients, and worked up to form granulate particles. The granulate is then mixed with the lubricant and, advantageously, with the second portion of the disintegrant to form a tablet blend. The lubricated granules are compressed into tablets. The tablets then may be coated to improve cosmetic and handling properties. The presence of microcrystalline cellulose gives a good option for direct compression or for dry granulation as well, particularly when also lactose is present in the composition.

The invention will be further described with reference to the following nonlimiting examples.

### Examples

Two batches of film-coated immediate release raloxifene tablets were made from the following compositions.

| | **Batch 1** | | **Batch 2** | |
|---|---|---|---|---|
| **Active/Excipient** | **Per tablet (mg)** | **(%)** | **Per tablet (mg)** | **(%)** |
| *Intra granular* | | | | |
| Raloxifene hydrochloride | 60 | 25.0 | 60 | 25.0 |
| Lactose monohydrate | 75.6 | 31.5 | 82.8 | 34.5 |
| MCC PH 101 | 72 | 30 | 72 | 30 |
| Crospovidone | 14.4 | 6.0 | 7.2 | 3.0 |
| HPC | 6.0 | 2.5 | 6.0 | 2.5 |
| Polysorbate 80 | 2.4 | 1.0 | 2.4 | 1.0 |

| *Extra granular* | | | | |
|---|---|---|---|---|
| Crospovidone | 7.2 | 3.0 | 7.2 | 3.0 |
| Mg stearate | 2.4 | 1.0 | 2.4 | 1.0 |
| Total uncoated tablet | 240 | 100 | 240 | 100 |

| *Coating* | | | | |
|---|---|---|---|---|
| Opadry white | 14.4 | 6 | 14.4 | 6 |
| Total coated tablet | 254.4 | 106 | 254.4 | 106 |

For each of Batches 1 and 2, a solution of hydroxypropylcellulose (HPC), Polysorbate 80 (Tween 80), and water was made and stirred overnight with a magnetic stirrer until a clear solution was obtained.

Raloxifene hydrochloride, lactose, microcrystalline cellulose (MCC), and crospovidone were put in the bowl of the granulator. The blend was stirred for 5 minutes with an impeller speed of 200 rpm and a chopper speed of 1500 rpm. The mixture was then granulated with an impeller speed of 235 rpm and chopper speed of 1500 rpm. The above solution was added to the blend at a speed of 55-60 g/min.

The granulate was dried in a drier, with an inlet temperature of 70°C, and milled through a 0.5 mm sieve. The granulate was then mixed with crospovidone for 15 min at 25 rpm in the free fall mixer. Magnesium stearate was sieved through a 0.85 mm sieve and mixed with the blend for 5 minutes at 25 rpm in the free fall mixer.

Tablets were compressed at the Korsch PH 106 tablet press with a tablet mass of 240 mg, a tablet diameter of 9 mm, and a hardness of 50-60N. The tablets were coated in a Bohle BCL 5 coater, with the following parameters: average inlet temp = 60-66 °C, spray rate = 5-15 g/min, average exhaust temp = 37-40°C, pan speed = 15 rpm and average bed temp= 49 °C.

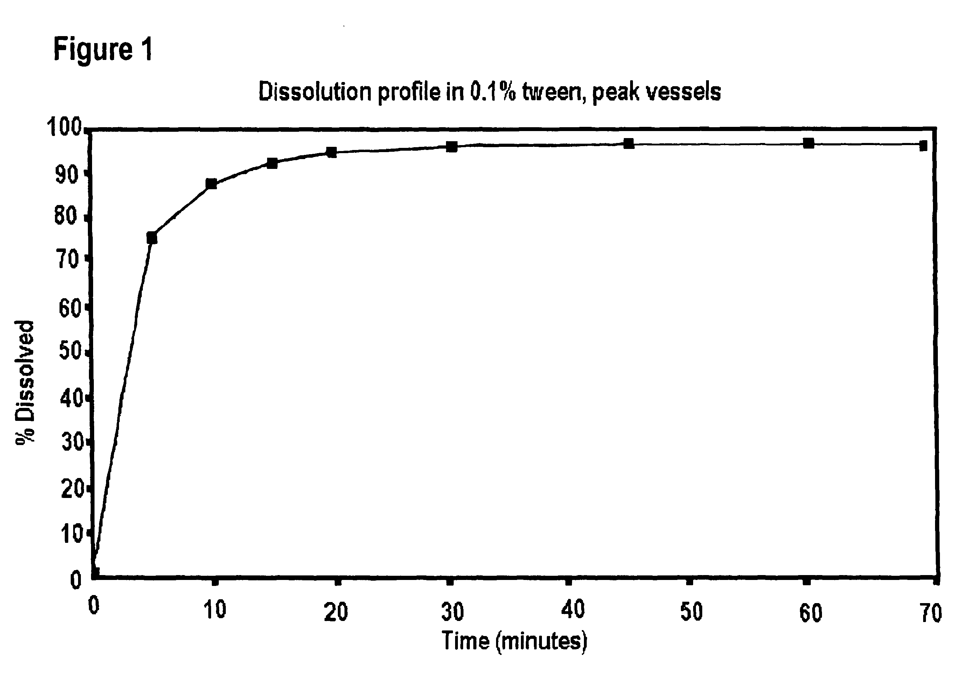

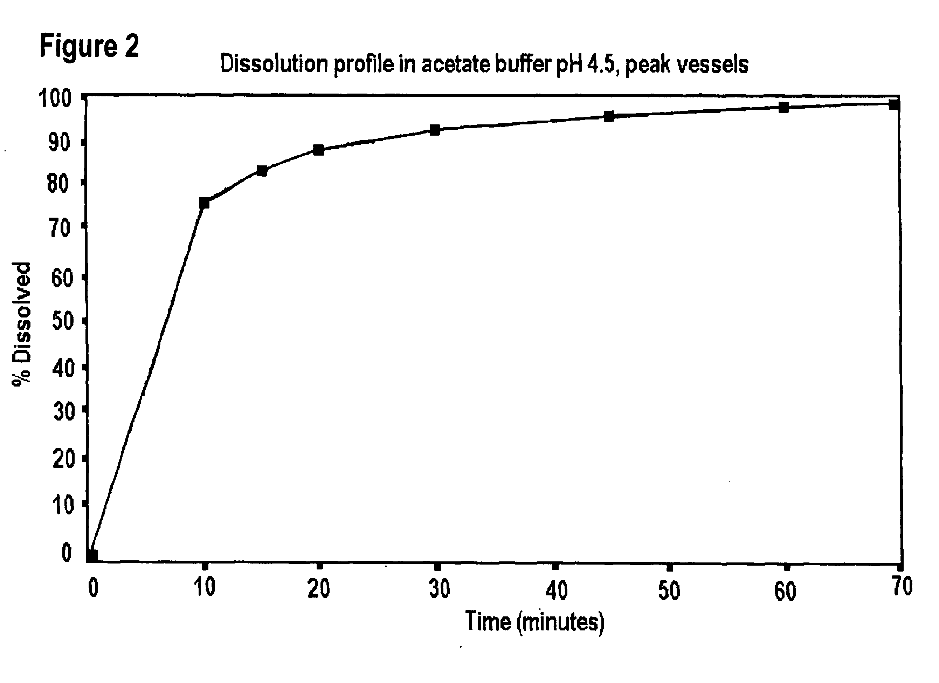

Dissolution profiles of the tablets made from Batch 2 are shown in Figure and Figure 2. Figure 1: Dissolution profile (USP apparatus II) was measured with three tablets at 1000 ml 0.1% Tween 80 solution at a rotation speed of 50 rpm in peak vessels and a temperature of 37.5°C. Figure 2: Dissolution profile (USP apparatus II) was measured with three tablets at 1000 ml acetate buffer pH 4.5 at a rotation speed of 50 rpm in peak vessels and a temperature of 37.5°C.

## Claims

1. A pharmaceutical composition, comprising:
a) raloxifene or a pharmaceutically acceptable salt thereof, preferably raloxifene hydrochloride, in an amount of 20 to 30 weight %;
b) a mixed cellulose excipient in an amount of 25 to 40 weight %, preferably 30 to 35 weight %, said mixed cellulose excipient containing 90 to 95 weight % microcrystalline cellulose and 5 to 10 weight % hydroxypropyl cellulose with respect to the total mass of the mixed cellulose excipient; and
c) a disintegrant in an amount of 5 to 12 weight %..

2. The pharmaceutical composition of claim 1, wherein said composition does not contain povidone or starch.

3. The pharmaceutical composition according to claim 1 or 2, which further comprises a sugar, preferably lactose monohydrate.

4. The pharmaceutical composition of claim 1-3, wherein said raloxifene is raloxifene hydrochloride having a mean particle size of 5 to 20 µm.

5. The pharmaceutical composition of claims 1-4, wherein 95% of the raloxifene hydrochloride particles are smaller than 50 µm.

6. The pharmaceutical composition according to any of claims 1-5, wherein said composition is an immediate release tablet.

7. The pharmaceutical composition of claim 6, wherein said tablet exhibits a disintegration time of less than five minutes in each of water and a simulated gastric fluid.

8. The pharmaceutical composition according to claim 6 or 7, wherein said tablet was made by wet granulation.

9. Pharmaceutical composition according to claim 1-8 for use in medicine, preferably for use in the treatment and/or prevention of osteoporosis.

## Patentansprüche

1. Pharmazeutische Zusammensetzung, umfassend:
a) Raloxifen oder ein pharmazeutisch verträgliches Salz davon, vorzugsweise Raloxifenhydrochlorid, in einer Menge von 20 bis 30 Gewichts-%;
b) einen gemischten Cellulosehilfsstoff in einer Menge von 25 bis 40 Gewichts-%, vorzugsweise 30 bis 35 Gewichts-%, wobei der gemischte Cellulosehilfsstoff 90 bis 95 Gewichts-% mikrokristalline Cellulose und 5 bis 10 Gewichts-% Hydroxypropylcellulose in Bezug auf die Gesamtmasse des gemischten Cellulosehilfsstoffs enthält; und
c) ein zerfallsförderndes Mittel in einer Menge von 5 bis 12 Gewichts-%.

2. Pharmazeutische Zusammensetzung nach Anspruch 1, wobei die Zusammensetzung kein Povidon oder Stärke enthält.

3. Pharmazeutische Zusammensetzung nach Anspruch 1 oder 2, das des Weiteren einen Zucker umfasst, vorzugsweise Laktosemonohydrat.

4. Pharmazeutische Zusammensetzung nach Anspruch 1-3, wobei das Raloxifen Raloxifenhydrochlorid mit einer mittleren Partikelgröße von 5 bis 20 µm ist.

5. Pharmazeutische Zusammensetzung nach Ansprüchen 1-4, wobei 95% der Raloxifenhydrochloridpartikel kleiner als 50 µm sind.

6. Pharmazeutische Zusammensetzung nach einem beliebigen der Ansprüche 1 - 5, wobei die Zusammensetzung eine unmittelbar freisetzende Tablette ist.

7. Pharmazeutische Zusammensetzung nach Anspruch 6, wobei die Tablette eine Zerfallszeit von weniger als fünf Minuten in jeweils Wasser und einer simulierten Magenflüssigkeit aufweist.

8. Pharmazeutische Zusammensetzung nach Anspruch 6 oder 7, wobei die Tablette durch feuchte Granulierung hergestellt wurde.

9. Pharmazeutische Zusammensetzung nach Anspruch 1-8, zur Verwendung in der Medizin, vorzugsweise zur Verwendung in der Behandlung und/oder der Prävention von Osteoporose.

## Revendications

1. Une composition pharmaceutique comprenant:
a) du raloxifène, ou un de ses sels pharmaceutiquement acceptable, de préférence le chlorhydrate de raloxifène, en une quantité de 20 à 30% en poids;
b) un excipient cellulosique mixte, en une quantité de 25 à 40% en poids, de préférence de 30 à 35% en poids, ledit excipient cellulosique mixte contenant de 90 à 95% en poids de cellulose microcristalline et de 5 à 10% en poids d'hydroxypropyl cellulose exprimés par rapport à la masse totale de l'excipient cellulosique mixte, et
c) un désintégrant en une quantité de 5 à 12% en poids.

2. La composition pharmaceutique selon la revendication 1, dans laquelle ladite composition ne contient ni polyvinylpyrrolidone ni amidon.

3. La composition pharmaceutique selon la revendication 1 ou 2, qui comprend en outre un sucre, de préférence du monohydrate de lactose.

4. La composition pharmaceutique selon les revendications 1 à 3, dans laquelle ledit raloxifène est du chlorhydrate de raloxifène présentant une dimension particulaire moyenne de 5 à 20 µm.

5. La composition pharmaceutique selon les revendications 1 à 4, dans laquelle 95% des particules de chlorhydrate de raloxifène sont plus petites que 50 µm.

6. La composition pharmaceutique selon les revendications 1 à 5, dans laquelle ladite composition est un comprimé à libération immédiate.

7. La composition pharmaceutique selon la revendication 6, dans laquelle ledit comprimé présente un temps de désintégration de moins de cinq minutes tant dans de l'eau que dans un fluide gastrique simulé.

8. La composition pharmaceutique selon la revendication 6 ou 7, dans laquelle ledit comprimé a été obtenu par granulation par voie humide.

9. Composition pharmaceutique selon les revendications 1 à 8, pour une utilisation en médecine, de préférence pour une utilisation pour le traitement et/ou la prévention de l'ostéoporose.
